# EUROPÄISCHE PATENTANMELDUNG

(11) **EP 4 843 554 A1**
(43) Veröffentlichungstag der Anmeldung: **25.11.2026**
(21) Anmeldenummer: 25177884.1
(22) Anmeldetag: 21.05.2025
(51) Int. Cl.: A61K 8/04, A61K 8/27, A61K 8/31, A61K 8/34, A61K 8/42, A61K 8/86, A61K 8/9794, A61Q 9/02

(54) **RASIERSCHAUMZUSAMMENSETZUNG, VERFAHREN ZU DEREN HERSTELLUNG UND VERWENDUNG**

(71) Anmelder: Aerochemica Dr. Deppe GmbH, 47906 Kempen (DE)
(72) Erfinder: Deppe, Marc, 47906 Kempen (DE); Bruder, Gregor, 47906 Kempen (DE)
(74) Vertreter: mepat Patentanwälte

(57) **Zusammenfassung**

Die vorliegende Erfindung stellt eine seifenfreie Rasierschaumzusammensetzung bereit, die zum Auftragen auf eine behaarte Hautpartie, zum Aufweichen von Haaren auf der behaarten Hautpartie, zur Bereitstellung eines Rasierklingen-Gleitfilms auf der Hautpartie ausgebildet ist. Die Rasierschaumzusammensetzung weist zumindest 1,5 bis 5 Gew.-% zumindest eines Fettalkoholpolyglycolethers aus einem Fettalkohol mit 9 bis 25 Kohlenstoffatomen und 3 bis 30 Ethylenoxideinheiten, 2,5 bis 9 Gew.-% einer Ölkomponente, die acyclische Kohlenwasserstoffe mit 11 bis 45 Kohlenstoffatomen aufweist, 1,5 bis 5 Gew.-% zumindest eines Fettalkohols mit 12 bis 25 Kohlenstoffatomen und einen die 100 Gew.-% ergänzenden Anteil an Wasser auf. Ferner werden ein Verfahren zur Herstellung der seifenfreien Rasierschaumzusammensetzung und deren Verwendung offenbart.

## Beschreibung

Die Erfindung betrifft eine Rasierschaumzusammensetzung, ein Verfahren zur Herstellung der Rasierschaumzusammensetzung sowie deren Verwendung.

Rasierpräparate wie Rasierschäume, Rasiergele, Rasiercremes oder Rasierseifen werden verwendet, um Haare aufzuweichen und einen Gleitfilm auf der Haut zu erzeugen, sodass die Rasierklinge bei der Nassrasur sanft über die Haut gleiten kann und Hautreizungen vermieden werden. Die Verwendung von Rasierpräparaten ermöglicht bei korrekter Durchführung eine angenehme und hautschonende Rasur, sodass eine glatte Haut ohne Rasurbrand oder eingewachsene Haare erreicht wird. Dazu gehört, dass die Rückstände des Rasierpräparats nach der Rasur mit Wasser von der Haut entfernt werden, die daraufhin mit einem Aftershave-Produkt desinfiziert und ggf. mit einer Feuchtigkeitspflege versorgt wird.

Die Rasierpräparate unterscheiden sich in der Art des Schaums und der Anwendung. Rasierseife wird mithilfe eines Rasierpinsels und etwas Wasser aufgeschäumt, wobei ein feinblasiger und dichter Schaum erzeugt wird, der lange stabil bleibt. Rasierschaum kommt durch ein Treibgas aufgeschäumt aus einer Aerosoldose und ist für den Anwender daher zeitsparend und bequem. Der Sprühschaum ist meist sehr feinblasig und stabil, teilweise so stabil, dass sich beim Verteilen Schaumflocken bilden, die eine lückenlose Benetzung der Haut verhindern. Rasiergel schäumt beim Verteilen auf der befeuchteten Haut auf, wobei der gebildete Schaum weniger feinporig und stabil ist als beim Rasierschaum. Rasiercreme hingegen schäumt nur wenig bis gar nicht. Oft werden Schäume bzw. schäumbare Präparate beim Rasieren bevorzugt, da durch den Schaum erkennbar ist, welche Partien bereits rasiert worden sind.

Üblicherweise enthalten Rasierpräparate Seifen, bzw. verseifte Fettsäuren, und sind daher alkalisch, wodurch der Säureschutzfilm der Haut angegriffen wird und die Haut stark austrocknet.

Eine nachschäumende Rasierzusammensetzung mit verbessertem Hautgefühl und verbesserten Schaumeigenschaften ist aus EP 1 704 897 A1 bekannt. Die Zusammensetzung, die mit einem nachschäumenden Treibmittel aufgeschäumt werden kann, enthält neben Wasser eine verseifte Fettsäure mit 10 bis 24 Kohlenstoffatomen, ein nichtionisches Tensid, ein wasserlösliches Polyol mit 3 bis 9 Kohlenstoffatomen, einen hydrophoben Coemulgator, der insbesondere aus Propylenglycol-, Gylcerin-, Sorbitanestern von Carbonsäuren mit 6 bis 22 Kohlenstoffatomen ausgewählt ist, ein verdickendes Polymer, das kein Polyethylenglycol darstellt und insbesondere aus Cellulose-Derivaten, Stärken, etc., Polyalkylenglycolethern und -estern ausgewählt ist, und ein Polyethylenglycol mit einem mittleren Molekulargewicht von mindestens 300.000 Dalton.

Aus EP 0 941 059 B1 ist ein seifenfreies Rasierpräparat bekannt, das beim Verteilen auf der Haut aufschäumt. Es umfasst neben Wasser 0,5 bis 50 Gew.-% eines amphoteren Tensids, ausgewählt aus Betainen und Sultainen; 0,01 bis 2 Gew.-% eines Celluloseethers zur Viskositätskontrolle; 0,01 bis 5 Gew.-% eines Polyethylenglycol-Esters mit 12 bis 22 Kohlenstoffatomen. Sämtliche dort angeführten Beispielformulierungen umfassen außerdem Natrium-Cocoyl-Isethionat (7,5 oder 5 Gew.-%) als zweites Tensid und Natrium-Lauroyl-Sarcosinat (1,5 Gew.-%) oder Ammonium-Laurethsulfat 2EO (10 Gew.-%) als drittes Tensid, zwei Feuchthaltemittel, Glycerin (3 Gew.-%) und Sorbitol (0,7 Gew.-%) und Vitamin E-Acetat als Moisturizer (0,1 Gew.-%). Weitere Inhaltsstoffe, die nicht in allen Beispielformulierungen enthalten sind, umfassen bis zu 10 Gew.-% Schaumstabilisatoren, Conditioner (0,5 Gew.-% Polyquaternium 7, 1 Gew.-% Polyethylen- oder Propylenglycol) Triethanolamin oder Zitronensäure zur pH-Werteinstellung, Konservierungsmittel, Natriumchlorid als weiteres Viskositätskontrollmittel, sowie Farb- und Duftstoffe.

Zur Herstellung wird ein Großteil des Wassers zu den Feuchthaltemitteln und Farbstoffen gegeben, ggf. ein erster Schaumstabilisator (Crosspolymer) hinzugegeben, und auf 185°F (85 °C) erhitzt und eine Stunde gerührt. Der Polyethylenglycol-Ester wird zugegeben und gut vermischt, und dann Triethanolamin. Bei 150 °F (65,56 °C) wird das zweite Tensid, dann das dritte Tensid und ein zweiter Schaumstabilisator (Aminoxid) zugegeben. Danach werden Conditioner, das aus Betainen und Sultainen ausgewählte amphotere Tensid und das restliche Wasser hinzugefügt, und auf 100 °F (37,78 °C) abkühlen gelassen. Zum Schluss werden Vitamin, Duft- und Konservierungsstoffe zugegeben.

Ausgehend von diesem Stand der Technik ist es Aufgabe der vorliegenden Erfindung, eine verbesserte seifenfreie Rasierschaumzusammensetzung bereitzustellen, die einen guten Gleitfilm für eine Rasierklinge schafft.

Diese Aufgabe wird durch eine Rasierschaumzusammensetzung mit den Merkmalen des Anspruchs 1 gelöst.

Die weitere Aufgabe einer einfachen Herstellung zur Bereitstellung der Rasierschaumzusammensetzung wird durch das Verfahren mit den Merkmalen des unabhängigen Anspruchs 8 gelöst.

Weiterbildungen der Rasierschaumzusammensetzung und des Verfahrens zu deren Herstellung sind in den jeweiligen Unteransprüchen ausgeführt.

Eine Verwendung der Rasierschaumzusammensetzung zum Rasieren einer behaarten Hautpartie wird mit den Merkmalen des unabhängigen Anspruchs 13 offenbart.

Gemäß einer ersten Ausführungsform weist die erfindungsgemäße seifenfreie Rasierschaumzusammensetzung, die zum Auftragen auf eine behaarte Hautpartie, zum Aufweichen von Haaren auf der behaarten Hautpartie, zur Bereitstellung eines Rasierklingen-Gleitfilms auf der Hautpartie ausgebildet ist, zumindest folgendes auf:
- 1,5 bis 5 Gew.-% zumindest eines Fettalkoholpolyglycolethers aus einem Fettalkohol mit 9 bis 25 Kohlenstoffatomen und 3 bis 30 Ethylenoxideinheiten,
- 2,5 bis 9 Gew.-% zumindest einer Ölkomponente, die acyclische Kohlenwasserstoffe mit 11 bis 45 Kohlenstoffatomen aufweist,
- 1,5 bis 5 Gew.-% zumindest eines Fettalkohols mit 12 bis 25 Kohlenstoffatomen und
- einen die 100 Gew.-% ergänzenden Anteil an Wasser aufweist.

Die erfindungsgemäße seifenfreie Rasierschaumzusammensetzung erlaubt vorteilhaft eine gute Verteilung auf der Haut und hat sehr gute Hautpflegeeigenschaften: Die Rasierschaumzusammensetzung lässt sich als eine Öl-in-Wasser-Emulsion darstellen, die entweder infolge Zerstäubung aus einer Sprühdose oder auch durch die Verteilung auf der Haut und dort durch Einwirkung der Hautwärme, durchaus kombiniert mit der Verteilungsbewegung durch Hand oder Pinsel nach dem Auftragen oder währenddessen aufschäumt. Der ausgebildete Schaum ist stabil, feinblasig, reichhaltig und cremig, mit gutem Spreit- und Benetzungsverhalten. Der verteilte Schaum stellt einen guten Gleitfilm für die Rasierklinge bereit. Gleichzeitig weist die Rasierschaumzusammensetzung eine besonders gute Hautverträglichkeit auf und eignet sich zur Hautpflege, d. h., dass die nach der Rasur auf der Haut verbleibenden Reste der Rasierschaumzusammensetzung nicht abgewaschen werden müssen, sondern auf der Haut verteilt werden können und zur Pflege und Beruhigung der Haut beitragen.

Nach einer weiteren Ausführungsform der erfindungsgemäßen Rasierschaumzusammensetzung weist diese
- 2 bis 4 Gew.-%, insbesondere 3 Gew.-%, des zumindest einen Fettalkoholpolyglycolethers,
- 3,5 bis 7 Gew.-%, insbesondere 5 Gew.-%, der Ölkomponente,
- 2 bis 4 Gew.-%, insbesondere 3 Gew.-%, des zumindest einen Fettalkohols,
auf.

Nach einer noch weiteren Ausführungsform der erfindungsgemäßen Rasierschaumzusammensetzung ist ferner zumindest ein Pflanzenextrakt in einem zur Hautpflege wirksamen Anteil enthalten. Bevorzugt handelt es sich dabei um einen Pflanzenextrakt aus Aloe-Vera (oder kurz: Aloe-Vera-Extrakt). Der zur Hautpflege wirksame Anteil des Pflanzenextrakts aus Aloe-Vera, der aus Aloe-Vera-Gel getrocknet als Pulver vorliegt, liegt in der Rasierschaumzusammensetzung bei 8 bis 15 Gew.-%, bevorzugt 9 bis 13 Gew.-%, insbesondere bei 10 bis 12 Gew.-%. Der aus Aloe-Vera-Gel erhaltene getrocknete bzw. gefriergetrocknete Pulverextrakt stellt ein 200:1 Konzentrat dar, sodass ein 100%iges Aloe-Vera-Gel aus 0,5 g Pulver pro 100 ml erhalten wird. Bei Verwendung anderer Extraktformen mit einer von der Pulverform abweichenden Konzentration kann der Anteil in der Rasierschaumzusammensetzung entsprechend der jeweiligen Konzentration angepasst werden.

Ferner kann nach noch einer weiteren Ausführungsform die erfindungsmäße Rasierschaumzusammensetzung folgendes enthalten:
- 0,5 bis 1,5 Gew.-%, bevorzugt 0,7 bis 1,3 Gew.-%, insbesondere 1 Gew.-%, Panthenol und/oder
- 1 bis 3 Gew.-%, bevorzugt 1,5 bis 2,2 Gew.-%, insbesondere 2 Gew.-%, Zinklactat oder 0,05 bis 1 Gew.-%, bevorzugt 0,1 bis 0,9 Gew.-%, eines Konservierungsmittels, das aus einer Gruppe ausgewählt ist, die Natriumbenzoat, Kaliumsorbat Benzylalkohol, Benzoesäure, und Mischungen davon, sowie ggf. andere mit nichtionischen Tensiden verträgliche Konservierungsstoffe umfasst, und/oder
- einen zur Einstellung eines bestimmten pH-Werts der Rasierschaumzusammensetzung in einem Bereich von 4 bis 8, bevorzugt von 5 bis 5,5, erforderlichen Anteil zumindest eines pH-Wert-Regulierers, der aus einer Gruppe ausgewählt ist, die Zitronensäure und

Milchsäure zur pH-Wert-Verringerung und Natriumhydroxyd zur pH-Wert-Erhöhung umfasst, und/oder
- bis zu 0,5 Gew.-% zumindest eines Duftstoffs.

Nach einer noch weiteren Ausführungsform der erfindungsgemäßen Rasierschaumzusammensetzung besteht diese vorteilhaft aus
- 1,5 bis 5 Gew.-% des zumindest einen Fettalkoholpolyglycolethers,
- 2,5 bis 9 Gew.-% der Ölkomponente,
- 1,5 bis 5 Gew.-% des zumindest einen Fettalkohols,
- 8 bis 15 Gew.-% des zumindest einen Pflanzenextrakts,
- 0,5 bis 1,5 Gew.-% Panthenol,
- 1 bis 3 Gew.-% Zinklactat oder 0,05 bis 1 Gew.-% des Konservierungsmittels,
- einem zur Einstellung eines bestimmten pH-Werts der Rasierschaumzusammensetzung in einem Bereich von 4 bis 8, bevorzugt von 5 bis 5,5, erforderlichen Anteil zumindest eines pH-Wert-Regulierers, der aus einer Gruppe ausgewählt ist, die Zitronensäure oder Milchsäure zur pH-Wert-Verringerung und Natriumhydroxyd zur pH-Wert-Erhöhung umfasst, und
- bis zu 0,5 Gew.-% des zumindest eines Duftstoffs, sowie
dem die 100 Gew.-% ergänzenden Anteil an Wasser.

Durch die begrenzte Anzahl der Inhaltsstoffe der erfindungsgemäßen Rasierschaumzusammensetzung wird das Risiko von Hautunverträglichkeitsreaktionen minimiert.

Nach noch einer weiteren bevorzugten Ausführungsformen der erfindungsgemäßen Rasierschaumzusammensetzung kann als Pflanzenextrakt ein Pflanzenextrakt aus Aloe Vera, getrocknet als Pulver, eingesetzt werden. Es kann Zinklactat zur Konservierung und Milchsäure zur pH-Einstellung verwendet werden, sodass die Rasierschaumzusammensetzung besonders gute Hautpflegeeigenschaften aufweist.

Nach einer weiteren Ausführungsform der erfindungsgemäßen Rasierschaumzusammensetzung besteht die Ölkomponente aus einer der folgenden Ölkomponenten, die jeweils acyclische Kohlenwasserstoffe mit 11 bis 45 Kohlenstoffatomen aufweisen:
- verzweigte, acyclische, gesättigte Kohlenwasserstoffe mit 15 bis 45 Kohlenstoffatomen, bevorzugt acyclische Terpen-Kohlenwasserstoffe, besonders bevorzugt Sesquiterpene oder Triterpene, insbesondere Squalan, oder
- paraffinische - also unverzweigte, acyclische, gesättigte - Kohlenwasserstoffe mit 15 bis 45, bevorzugt 18 bis 32, Kohlenstoffatomen, insbesondere Paraffinöl Perliquidum, oder

- eine Mischung aus paraffinischen Kohlenwasserstoffen mit 11 bis 25, bevorzugt 15 bis 19, Kohlenstoffatomen, und verzweigten, acyclischen Kohlenwasserstoffen mit 15 bis 45 Kohlenstoffatomen, die bevorzugt acyclischen Terpen-Kohlenwasserstoffe, besonders bevorzugt Sesquiterpene oder Triterpene sind,
   oder
- eine Mischung aus paraffinischen Kohlenwasserstoffen mit 11 bis 25, bevorzugt mit 15 bis 19, Kohlenstoffatomen und Fettsäureestern aus Fettsäuren mit 8 bis 20 Kohlenstoffatomen und Fettalkoholen mit 6 bis 25 Kohlenstoffatomen.

Squalan als Ölkomponente ist sehr vorteilhaft.

Nach einer noch weiteren Ausführungsformen der erfindungsgemäßen Rasierschaumzusammensetzung besteht der zumindest eine Fettalkoholpolyglycolether aus einem Fettalkohol mit 14 bis 22, bevorzugt 16 bis 20, insbesondere 18, Kohlenstoffatomen mit 4 bis 30, bevorzugt 10 bis 30, besonders bevorzugt 15 bis 25, insbesondere 21, Ethylenoxideinheiten. Zusätzlich oder alternativ kann der zumindest eine Fettalkohol 14 bis 22, insbesondere 16 bis 18, Kohlenstoffatome aufweisen.

Gemäß einer ersten Ausführungsform des Verfahrens zu Herstellung der seifenfreien Rasierschaumzusammensetzung umfasst dieses die Schritte des Zusammengebens des zumindest einen Fettalkoholpolyglycolethers, der Ölkomponente und des zumindest einen Fettalkohols, Erhitzen auf eine erste Temperatur in einem Bereich von 75 bis 85 °C, insbesondere 80 °C, und des Durchmischens des zumindest einen Fettalkoholpolyglycolethers, der Ölkomponente und des zumindest einen Fettalkohols durch Rühren. Weiter erfolgt das Erhitzen des Wassers auf die erste Temperatur, und in das erhitze Wasser wird die erhitzte Mischung aus dem zumindest einen Fettalkoholpolyglycolethers, der Ölkomponente und des zumindest einen Fettalkohols zugegeben. Es erfolgt das Homogenisieren zu einer Emulsion und das Abkühlen der Emulsion.

Nach einer weiteren Ausführungsform des erfindungsgemäßen Verfahrens erfolgt das Abkühlen der Emulsion durch:
- Abkühlen der Emulsion auf eine zweite Temperatur in einem Bereich von 35 bis 45 °C, insbesondere auf 40 °C,
- Zugeben des zumindest einen Pflanzenextrakts, des Panthenols, des Zinklactats oder des Konservierungsmittels, des zumindest einen pH-Wert-Regulierers und des zumindest einen Duftstoffs zu der auf 40 °C abgekühlten Emulsion,
- erneut Homogenisieren und Abkühlen auf eine dritte Temperatur in einem Bereich der Raumtemperatur, bevorzugt in einem Bereich von 15 bis 25 °C, insbesondere auf 20 °C.

Das Homogenisieren kann dabei nach einer weiteren Ausführungsform des erfindungsgemäßen Verfahrens jeweils unter Unterdruck im Grobvakuum, bevorzugt bei einem Druck im Bereich um 500 hPa, erfolgen.

Ferner kann das erfindungsgemäße Verfahren nach noch einer weiteren Ausführungsform das Abfüllen einer vorbestimmten Menge der Rasierschaumzusammensetzung in einen Behälter umfassen, bevorzugt in einen Behälter, der aus einer Gruppe ausgewählt ist, die eine Sprühdose, einen Ventilbeutelbehälter und einen luftlosen Pumpspender auf-weist, oder in einen anderen Behälter, in dem die abgefüllte Rasierschaumzusammensetzung während des Gebrauchs vor mikrobiellem Befall geschützt ist.

Je nach Auswahl des Behälters, ob Sprühdose oder Ventilbeutelbehälter oder luftloser Pumpspender - umfassen weitere Ausführungsformen des erfindungsgemäßen Verfahrens die Schritte
- Verbringen von Treibgas in die Sprühdose nach dem Abfüllen der vorbestimmten Menge der Rasierschaumzusammensetzung in die Sprühdose, wobei das Treibgas aus der Gruppe ausgewählt ist, die Propan, Butan, Isopentan, Isobutan, Stickstoff, Kohlensäure und Mischungen davon umfasst, wobei das Treibgas einen Anteil von bis zu 5 Gew.-%, bevorzugt 3 bis 4 Gew.-% der treibgashaltigen Rasierschaumzusammensetzung ausmacht, oder
- Anordnen eines Ventilbeutels in dem Ventilbeutelbehälter und Verbringen eines Treibmittels in den Ventilbeutelbehälter vor dem Einfüllen der vorbestimmten Menge der Rasierschaumzusammensetzung in den Ventilbeutel, wobei das Treibmittel bevorzugt ein nicht brennbares Treibgas ist, oder
- luftdicht Anordnen einer Kolbenplatte in dem Behälter eines luftlosen Pumpspenders vor oder nach dem Abfüllen der vorbestimmten Menge der Rasierschaumzusammensetzung in den Behälter.

Eine erfindungsgemäße Verwendung der seifenfreien Rasierschaumzusammensetzung zum Rasieren einer behaarten Hautpartie umfasst das Aufschäumen der Rasierschaumzusammensetzung unter Auftragen auf die behaarte Hautpartie, aufweichen Lassen von Haaren der behaarten Hautpartie und Bereitstellen des Rasierklingen-Gleitfilms auf der Hautpartie. Dabei lässt sich die Rasierschaumzusammensetzung außerdem vorteilhaft einfach durch Verteilen von Resten der Rasierschaumzusammensetzung, die nach der Rasur auf der Hautpartie verbleiben, zur Hautpflege verwenden.

Weitere Ausführungsformen der erfindungsgemäßen Rasierschaumzusammensetzung und des Verfahrens sowie einige der Vorteile, die mit diesen und weiteren Ausführungsformen der Rasierschaumzusammensetzung und des Herstellungsverfahrens verbunden sind, werden durch die nachfolgende ausführliche Beschreibung deutlich und besser verständlich.

Die nachfolgende Tabelle 1 zeigt ein Beispiel für eine erfindungsgemäße Rasierschaumzusammensetzung, die z. B. als treibgashaltige Rasierzusammensetzung mit bis zu 5 Gew.-%, beispielsweise 3 bis 4 Gew.-% Treibgas in einer Sprühdose bereitgestellt werden kann:

**Tabelle 1: Beispiel für eine erfindungsgemäße Rasierschaumzusammensetzung**

| Komponente | Funktion | Beispiel | Gew.-% |
|---|---|---|---|
| Fettalkoholpolyglycolether | Tensid/Emulgator | Steareth-21 | 3,0 |
| Ölkomponente | Ölkomponente | Squalan | 5,0 |
| Fettalkohol | Emulsionsstabilisator | Cetylstearylalkohol | 3,0 |
| Pflanzenextrakt | Hautpflege | Aloe-Vera-Extrakt | 10,0 |
| Panthenol | Hautpflege | | 1,0 |
| Zinklactat | Konservierung | | 2 |
| Milchsäure | pH-Wert-Einstellung | für pH 5,5 | 0,1 |
| Duftstoff | Parfümierung | ggf. | bis 0,5 |
| | | | |
| Wasser demineralisiert | | | ad 100 |

Diese Rasierschaumzusammensetzung wurde einem Konservierungs-Belastungs-Test nach ISO 11930:2022-08 unterzogen, bei dem die Keimzahlentwicklung bei 20-25°C nach Beimpfen der Probe mit den in Tabelle 2 aufgeführten Keimsuspensionen beobachtet wird.

**Tabelle 2: Konservierungs-Belastungs-Tests an einer Rasierschaumzusammensetzung nach Beispiel 1 mit 2 Gew.-% Zinklactat und einem pH-Wert von 5,5.**

| Untersuchungen | 0 h | 7 d | 14 d | 28 d |
|---|---|---|---|---|
| Pseudomonas paraeruginosa (früher Pseudomonas aeruginosa) ATCC 9027 | 4,5×10⁵ | 6,2×10³ | < 1,0×10¹ | < 1,0×10¹ |
| Logarithmische Reduktionswerte | - | 1,9 | > 4,7 | > 4,7 |
| Staphylococcus aureus ATCC 6538 | 3,1×10⁵ | < 1,0×10¹ | < 1,0×10¹ | < 1,0×10¹ |
| Logarithmische Reduktionswerte | - | > 4,5 | > 4,5 | > 4,5 |
| Escherichia coli ATCC 8739 | 3,6×10⁵ | < 1,0×10¹ | < 1,0×10¹ | < 1,0×10¹ |
| Logarithmische Reduktionswerte | - | > 4,6 | > 4,6 | > 4,6 |
| Candida albicans ATCC 10231 | 3,2×10⁴ | 8,1×10³ | < 1,0×10¹ | < 1,0×10¹ |
| Logarithmische Reduktionswerte | - | 0,6 | > 3,5 | > 3,5 |
| Aspergillus brasiliensis ATCC 16404 | 3,5×10⁴ | - | < 1,0×10¹ | < 1,0×10¹ |
| Logarithmische Reduktionswerte | - | - | > 3,5 | > 3,5 |

Alle Keimzahlen sind ausgedrückt in KBE/g bzw. ml (KBE = Koloniebildende Einheiten)

Die Bewertungskriterien für die Belastungstests (logarithmische Reduktionswerte) nach DIN EN ISO 11930:2022-08 (eine Abweichung von 0,5 log-Stufen ist zulässig) sind in Tabelle 3 angegeben.

Tabelle 3: Bewertungskriterien der Konservierungs-Belastungs-Tests an der Rasierschaumzusammensetzung nach Beispiel 1 mit 2 Gew.-% Zinklactat und einem pH-Wert von 5,5.

| | 0 h | 7d | 14 d | 28 d |
|---|---|---|---|---|
| Bakterien (Kriterium A) | - | ≥ 3,0 | ≥ 3,0 * | ≥ 3,0 * |
| Bakterien (Kriterium B) | - | - | ≥ 3,0 | ≥ 3,0 * |
| Candida albicans (Kriterium A) | - | ≥ 1,0 | ≥ 1,0 * | ≥ 1,0 * |
| Candida albicans (Kriterium B) | - | | ≥ 1,0 | ≥ 1,0 * |
| Aspergillus brasiliensis (Kriterium A) | - | | ≥ 0,0 | ≥ 1,0 * |
| Aspergillus brasiliensis (Kriterium B) | - | | ≥ 0,0 | ≥ 0,0* |

| | | | | |
|---|---|---|---|---|
| * kein Anstieg seit der letzten Zählung | | | | |

Die Gesamtanzahl aerober mesophiler Bakterien pro Gramm, bestimmt nach ISO 21149:2022-08, lag bei < 10, und die Gesamtanzahl an Hefen und Schimmelpilzen pro Gramm, bestimmt nach ISO 16212:2022-08, lag bei 90, wobei alle Keimzahlen in KbE (Koloniebildende Einheiten) ausgedrückt sind.

Damit erfüllt die Rasierschaumzusammensetzung nach Beispiel 1 die Anforderungen nach Kriterium B des Konservierungs-Belastungs-Tests nach DIN EN ISO 11930:2022-08. Durch Abfüllung der Rasierschaumzusammensetzung in einem Behälter wie einer Sprühdose mit Treibgas, einem Ventilbeutelbehälter mit Treibmittel oder einen luftlosen Pumpspender kann die Rasierschaumzusammensetzung vor mikrobiellem Befall während des Gebrauchs geschützt werden.

In einer Sprühdose (auch Spray- oder Aerosoldose) steht die Rasierschaumzusammensetzung durch Treibgas unter Druck, wobei als Treibgas Propan, Butan, Isopentan, Isobutan, Stickstoff, Kohlensäure, ggf. auch Dimethylether, und Mischungen davon verwendet werden. Zur Entnahme der Rasierschaumzusammensetzung wird der Sprühkopf gedrückt, sodass die Rasierschaumzusammensetzung bei der Freisetzung mit dem Treibgas aus der Düse aufgeschäumt wird.

In einem Ventilbeutelbehälter (auch Bag-on-Valve-System) liegt die Rasierschaumzusammensetzung von dem Treibmittel getrennt vor. Dazu wird ein Beutel, der mit einem Ventil verbunden ist, in den Behälter eingebracht, der mit dem Ventil verbunden und mit Treibmittel unter Druck gesetzt wird. Darauf wird das Produkt in den Beutel verbracht, wobei das Treibmittel den für die Entnahme der Rasierschaumzusammensetzung notwendigen Druck bereitstellt.

In einem luftlosen Pumpspender (auch Airless-Spender) ist im Behälter eine bewegliche Kolbenplatte luftdicht angeordnet, sodass sie in dem Behälter einen mit dem Ventil des Spenderkopfes verbundenen Füllbereich von einem Luftbereich trennt, der durch den Behälterboden begrenzt wird. Der Spenderkopf mit dem Ventil und der Membran verschließt eine Spenderöffnung auf einer Seite des Behälters gegenüber dem Behälterboden. Beim Drücken des Spenderkopfes wird mittels einer Membran ein Vakuumeffekt erzeugt, durch den die Kolbenplatte in Richtung des Spenderkopfs bewegt wird und die Rasierschaumzusammensetzung durch dessen Ventil drückt. Dabei strömt Umgebungsluft in den Luftbereich des Behälters durch eine entsprechend in der Behälterwand vorliegende Luftöffnung. Diese kann z. B. in dem Behälterboden sein.

All diese Behälter ermöglichen eine hygienische Entnahme der Rasierschaumzusammensetzung, bei der keine Keime ins Innere des Behälters gelangen können. Des Weiteren ist die Rasierschaumzusammensetzung in diesen Behältern vor Oxidation und anderen Einflüssen von außen geschützt.

Die seifenfreie, cremige Rasierschaumzusammensetzung lässt sich als Öl-in-Wasser-Emulsion ferner in einem einfachen Verfahren herstellen. Dabei werden der Fettalkoholpolyglycolether, die Ölkomponente und der Fettalkohol, zusammengegeben und unter Rühren bis zur vollständigen Verflüssigung, vorzugsweise auf 80 °C erhitzt und durchmischt, um eine Öl-Phase zu bilden. Die erhitzte Öl-Phase wird in das auf ebenfalls 80 °C erhitzte demineralisierte Wasser gegeben und zu einer Emulsion homogenisiert, die in einer ersten Kühlstufe, vorzugsweise auf 40 °C abgekühlt wird. Die restlichen Inhaltsstoffe, d. h. der Pflanzenextrakt, das Panthenol, Zinklactat, Milchsäure zur pH-Wert-Einstellung und ggf. Duftstoff werden zu der auf 40 °C abgekühlten Emulsion hinzugegeben, worauf erneut homogenisiert wird. Die so erhaltene Rasierschaumzusammensetzung wird dann in einer zweiten Kühlstufe auf Raumtemperatur bzw. 20 °C gekühlt.

Zum Homogenisieren im Labormaßstab bei Umgebungsdruck wurde ein handelsüblicher Homogenisator verwendet. Im Produktionsmaßstab wurde eine Vakuum-Misch-Homogenisierungsanlage 2000L der Firma Hebold verwendet, wobei das Mischen und Homogenisieren bei mittlerer Intensität unter Unterdruck im Grobvakuum, beispielsweise bei einem Druck von ca. 500 hPa erfolgt.

Um die Rasierschaumzusammensetzung anwendungsfertig für den Verbraucher bereitzustellen, umfasst das Verfahren außerdem die Abfüllung der Rasierschaumzusammensetzung in Behälter wie die vorgenannten Sprühdosen, Ventilbeutelbehälter und luftlosen Pumpspender, in denen die abgefüllte Rasierschaumzusammensetzung vor mikrobiellem Befall geschützt ist.

Je nach Art des Behälters unterscheidet sich das Abfüllen entsprechend, wobei zum Abfüllen der Rasierschaumzusammensetzung in Sprühdosen zuerst eine vorbestimmte Menge der Rasierschaumzusammensetzung in jede Dose verbracht wird. Dann werden die Dosen mit einem Ventil verschlossen, bevor das Treibgas eingefüllt wird, das beim Abfüllen bis zu 5 Gew.-%, bevorzugt 3 bis 4 Gew.-% des Doseninhalts ausmacht.

Werden Ventilbeutelbehälter verwendet, umfasst das Abfüllen der Rasierschaumzusammensetzung zunächst das Einbringen eines Ventilbeutels in jeden Behälter, das Verbinden des Ventils mit dem Behälter und das Einfüllen eines Treibmittels, vorzugsweise eines nicht brennbaren Treibgases, z. B. Druckluft, in jeden Behälter, bevor eine vorbestimmte Menge der Rasierschaumzusammensetzung in jeden Ventilbeutel verbracht wird.

Das Abfüllen der Rasierschaumzusammensetzung in luftlose Pumpspender variiert je nach Bauart des luftlosen Pumpspenders, insbesondere in Bezug auf die Lage der Luftöffnung, durch die Luft in den Luftbereich einströmen kann. Und auch wenn die Luftöffnung in dem Behälterboden vorliegt, gibt es zwei Varianten des Abfüllens: In einer Variante ist der Behälterboden einstückig mit dem Behälter verbunden, wobei das Abfüllen der Rasierschaumzusammensetzung durch die Spenderöffnung erfolgt, nachdem die Kolbenplatte in dem Behälter luftdicht angeordnet worden ist. Die Kolbenplatte wird entweder vor dem Abfüllen oder vorzugsweise zur Vermeidung von Lufteinschlüssen während des Abfüllens durch Druck der abgefüllten Rasierschaumzusammensetzung in Richtung des Behälterbodens geschoben. Nach dem Abfüllen wird die Spenderöffnung durch Anordnung des Spenderkopfs am Behälter verschlossen. In der zweiten Variante wird der Behälterboden durch ein Bodenelement gebildet, mit dem eine Füllöffnung des Behälters verschlossen werden kann, dessen Spenderöffnung bereits durch den Spenderkopf verschlossen ist. Dabei wird die Rasierschaumzusammensetzung durch die Füllöffnung in den Behälter abgefüllt, bevor die Kolbenplatte luftdicht in den Behälter angeordnet und danach das Bodenelement mit dem Behälter verbunden wird. Bei beiden Varianten können beim Abfüllen ggf. eingebrachte Lufteinschlüsse im Behälter durch Pumpen mittels Betätigung des Spenderkopfes aus dem Füllbereich entfernt werden.

Je nach Art des Behälters unterscheidet sich bei der Verwendung der seifenfreien Rasierschaumzusammensetzung zum Rasieren einer behaarten Hautpartie die Entnahme bzw. Ausgabe einer Portion der Rasierschaumzusammensetzung und deren Aufschäumen. Bei Sprühdosen schäumt die bei Betätigung des Sprühkopfes durch dessen Düse austretende treibgashaltige Rasierschaumzusammensetzung von selbst auf. Bei luftlosen Pumpspendern tritt die Rasierschaumzusammensetzung bei Betätigung des Spenderkopfes als ungeschäumtes Rasiergel aus, das unter Einwirkung der Hautwärme in den Händen oder beim Verteilen auf der Hautpartie aufschäumt. Ventilbeutelbehälter ermöglichen durch Einstellung des Treibmitteldrucks und Auswahl des Sprühkopfes sowohl die Ausgabe der Rasierschaumzusammensetzung als Rasierschaum als auch als Rasiergel.

Unabhängig von der Art des Behälters umfasst die Verwendung der seifenfreien Rasierschaumzusammensetzung zum Rasieren einer behaarten Hautpartie das Auftragen der aufgeschäumten Rasierschaumzusammensetzung auf die behaarte Hautpartie, sodass die Rasierschaumzusammensetzung die Haare aufweicht und einen Rasierklingen-Gleitfilms auf der Hautpartie bereitstellt. Nach erfolgter Rasur können die auf der Hautpartie verbleibenden Reste der Rasierschaumzusammensetzung zur Hautpflege verwendet und einmassiert werden und müssen nicht abgewaschen werden.

Denn vorteilhaft weist die erfindungsgemäße Rasierschaumzusammensetzung nur wenige Inhaltsstoffe auf, wodurch das Risiko von Hautunverträglichkeitsreaktionen minimiert wird. Im Gegenteil wirkt die Rasierschaumzusammensetzung mit Squalan, Aloe-Vera-Extrakt, Panthenol sogar anti-irritativ, und anti-entzündlich, pflegend und beruhigend.

Im oben in Tabelle 1 angeführten Beispiel wird als Fettalkoholpolyglycolether Steareth-21 (CAS: Nr. 9005-00-9) eingesetzt, ein Polyethylenglycoloctadecylether, der aus Stearylalkohol mit durchschnittlich 21 Ethylenoxideinheiten (-CH₂-CH₂-O-) gebildet wird.

Alternativ dazu einsetzbare Fettalkoholpolyglycolether sind z. B. Polyethylenglycoloctadecylether, die aus Stearylalkohol (C18) mit einer von 21 abweichenden Anzahl Ethylenoxideinheiten gebildet werden, und Fettalkoholpolyglycolether, die aus einem anderen Fettalkohol, wie z. B. Cetylalkohol (C16) oder auch Laurylalkohol (C12), Myristylalkohol (C14), Margarylalkohol (C17), Arachidylalkohol (C20), Behenylalkohol (C22) oder Lignocerylalkohol (C24) mit unterschiedlichen Anzahlen Ethylenoxideinheiten gebildet. Es können auch Mischungen aus verschiedenen Fettalkoholpolyglycolethern eingesetzt werden, wenngleich es bevorzugt ist, die Anzahl der Inhaltsstoffe in der Rasierschaumzusammensetzung so gering wie möglich zu halten. Daher wird in der Rasierschaumzusammensetzung bevorzugt nur ein Fettalkoholpolyglycolether eingesetzt.

Als bevorzugte Ölkomponente wird Squalan (2,6,10,15,19,23-Hexamethyltetracosan; CAS-Nr.111-01-3) in der Rasierschaumzusammensetzung eingesetzt, ein farb- und geruchloses, oxidationsstabiles Öl, das ursprünglich aus Haileber und mittlerweile auch aus pflanzlichen Quellen wie z. B. Oliven oder Zuckerrohr gewonnen wird. Squalan ist ein acyclischer, gesättigter Triterpen-Kohlenwasserstoff mit der Summenformel C₃₀H₆₂, der aus drei Terpeneinheiten oder zwei Farneseneinheiten, also insgesamt sechs Isopreneinheiten, gebildet ist. Mit Squalan als Ölkomponente der Rasierschaumzusammensetzung wird die Hydratation, Geschmeidigkeit und die Weichheit der Haut verbessert, und gleichzeitig eine Hautschutzbarriere gebildet.

Zu Squalan alternative Ölkomponenten, die in der Rasierschaumzusammensetzung einsetzbar sind, sind beispielsweise andere acyclische Terpen-Kohlenwasserstoffe mit 15 bis 45 Kohlenstoffatomen, die aus Terpen-, bzw. Farnesen- oder Isopreneinheiten gebildet werden, wie beispielsweise hydrierte Polyfarnesene, die aus zwei oder mehr Farneseneinheiten gebildet sind.

Eine weitere, alternativ einsetzbare Ölkomponente ist Paraffinöl perliquidum, ein dünnflüssiges Paraffinölgemisch aus acyclischen, gesättigten Kohlenwasserstoffen, auch medizinisches, hochreines Weißöl (CAS-Nr. 8042-47-5) genannt. Dieses Paraffinölgemisch, das bei 20°C eine relative Dichte von 0,81 bis 0,89 g/cm³ und eine dynamische Viskosität von 25 bis 80 mPa·s aufweist, hat wie Squalan filmbildende und schützende Eigenschaften und wirkt in der Rasierschaumzusammensetzung als Konsistenzbildner, kann aber als Mineralölderivat weniger bevorzugt sein.

Ferner ist es möglich, als Ölkomponente eine Mischung aus unterschiedlichen acyclischen, gesättigten Kohlenwasserstoffen als Emolliens zur Verbesserung der Geschmeidigkeit und Weichheit der Haut einzusetzen, z. B. eine Mischung aus paraffinischen und Terpen-Kohlenwasserstoffen. Ein Beispiel für eine solche Mischung weist hochreine und biologisch abbaubare C15-C19 Alkane und hydriertes Polyfarnesen auf und wird unter dem Handelsnamen EMOGREEN^{™} HP 40 von Seppic S.A., Frankreich, vertrieben.

Eine weitere alternative Ölkomponente ist eine Mischung aus acyclischen, gesättigten Kohlenwasserstoffen und Fettsäure-Estern. Ein Beispiel dafür ist eine Mischung aus hochreinen und biologisch abbaubaren C15-C19 Alkanen und Coco-Caprylate/Caprate (englische bzw. INCI-Bezeichnung, deutsche Bezeichnung: Kokos-Caprate/-Caprinate) und wird unter dem Handelsnamen EMOGREEN^{™} C69 von Seppic S.A., Frankreich, vertrieben. Bei diesen Fettsäure-Estern handelt es sich um Ester der Caprylsäure (C8) und Caprinsäure (C10) mit Fettalkoholen aus Kokos-Fettsäuren, die insbesondere C8-C18 Fettsäuren umfassen.

Beide Mischungen sind stabil, geruchsneutral und farblos und verbinden eine gute Verteilbarkeit auf der Haut und filmbildende Eigenschaften mit einem weichen Hautgefühl.

Der in der Rasierschaumzusammensetzung des Beispiels in Tabelle 1 eingesetzte Fettalkohol ist Cetylstearylalkohol (CAS: 67762-27-0), ein wachsartiges Gemisch aus hauptsächlich Stearylalkohol (C18) und Cetylalkohol (C16). Alternativ zu dem Gemisch kann als Fettalkohol in der Rasierschaumzusammensetzung Stearylalkohol oder Cetylalkohol eingesetzt werden. Weitere einsetzbare Fettalkohole umfassen beispielsweise Laurylalkohol (C12), Myristinalkohol (C14), Arachidylalkohol (C20), Behenylalkohol (C22), Lignocerylalkohol (C24) sowie Mischungen davon oder mit Stearylalkohol oder Cetylalkohol.

Fettalkohole sind nichtionische Tenside mit viskositätsmodifizierenden und filmbildenden Eigenschaften und werden als Emulsionsstabilisator und Conditioner eingesetzt. Dadurch wird das Schäumen unterstützt, die Konsistenz und Stabilität des Schaums und die Verteilbarkeit verbessert, und durch Bildung einer Schutzschicht der Feuchtigkeitsverlust der Haut reduziert.

Der in der Beispielzusammensetzung in Tabelle 1 eingesetzte Pflanzenextrakt ist ein Aloe-Vera-Extrakt, der aus dem wirkstoffhaltigen Wasserspeichergewebe (Parenchym) im Inneren der Blätter gewonnen wird. Das aus dem wirkstoffhaltigen Wasserspeichergewebe erhaltene Aloe-Vera-Gel besteht bis zu 99,5% aus Wasser und enthält Polysaccharide, insbesondere Acemannan, ein acetyliertes Polysaccharid, Vitamine, organische Säuren und Glucose. Der Pflanzenextrakt aus Aloe-Vera verleiht der Rasierschaumzusammensetzung feuchtigkeitsspendende, hautberuhigende, wundheilende und regenerative Eigenschaften, die hauptsächlich auf das in Aloe-Vera enthaltene Acemannan zurückgeführt werden.

Bei dem in der Rasierschaumzusammensetzung in Tabelle 1 eingesetzten Aloe-Vera-Extrakt handelt es sich um ein aus dem Aloe-Vera-Gel hergestelltes (gefrier-)getrocknetes Pulver, das ein 200:1 Konzentrat darstellt, d. h., dass 0,5 g Pulver auf 100 ml destilliertes Wasser 100%iges Aloe-Vera-Gel ergeben. Vorteilhaft wirkt sich der Aloe-Vera-Extrakt in Pulverform positiv auf die Schaumstabilität aus.

Alternativ zu dem bevorzugt als Pflanzenextrakt eingesetzten Aloe-Vera-Pulver kann als Pflanzenextrakt direkt das aus Aloe-Vera gewonnene Gel, ein Öl-Extrakt aus Aloe-Vera oder ein Aloe-Vera-Extrakt mit einem anderen Lösungsmittel eingesetzt werden. Da sich die vorliegenden Anteilsangaben für den Aloe-Vera-Pflanzenextrakt auf dessen Pulverform, d. h. das 200:1 Konzentrat beziehen, ist bei Verwendung alternativer Extraktformen der Anteil in der Rasierschaumzusammensetzung an die jeweilige Konzentration anzupassen.

Grundsätzlich ist es auch möglich, dass die Rasierschaumzusammensetzung alternativ oder zusätzlich zu dem Aloe-Vera-Extrakt einen oder mehrere Pflanzenextrakte aus anderen Aloen, wie z. B. Aloe Arborescens, oder aus anderen Pflanzen in einem zur Hautpflege wirksamen Anteil aufweist, beispielsweise können Pflanzenextrakte aus Gurke, Melone, Kaktus, Arnika, Calendula, Centella, Augentrost, Hamamelis, Grüntee, Chiasamen, Granatapfelsamen etc. eingesetzt werden.

Das in der Rasierschaumzusammensetzung enthaltene Panthenol (Provitamin B5) trägt ebenfalls zur Hautpflege bei, da Panthenol entzündungshemmend und juckreizlindernd wirkt, den Feuchtigkeitsverlust durch Verbesserung der Hautschutzbarriere reduziert, und überdies die Wundheilung fördert, was besonders hilfreich ist, wenn die Haut durch die Rasierklinge verletzt wird.

Panthenol wird wegen seiner Vitaminfunktion bevorzugt eingesetzt, wobei Panthenol im Hautgewebe zu Pantothensäure (Vitamin B5) umgewandelt wird, die bei der Biosynthese des Coenzyms A beteiligt ist. Alternativ zu Panthenol oder zusätzlich dazu können der Rasierschaumzusammensetzung auch andere bei der Hautpflege einsetzbare (Pro-) Vitamine bzw. Wirkstoffe mit Vitaminfunktion oder Wirkstoffe ohne Vitaminfunktion wie Urea, Manuka, Zinkoxid zur Unterstützung von Heilungsprozessen zugesetzt werden.

Grundsätzlich ist es möglich, dass die Rasierschaumzusammensetzung Konservierungsstoffe wie Benzylalkohol, Benzoesäure und Mischungen davon aufweisen kann, um die Abfüllung in einen einfachen Behälter wie Tuben und Tiegel zu gestatten, bei denen die abgefüllte Rasierschaumzusammensetzung nach Anbruch mit Keimen aus der Luft oder der Haut in Kontakt kommen kann.

Ein geeignetes Konservierungsmittel im sauren pH-Wert-Bereich bis pH 5,4 ist beispielsweise Rokonsal^{™} BS, eine Mischung aus Natriumbenzoat und Kaliumsorbat, vertrieben durch die Firma Ashland LLC, Covington, Kentucky, USA. Dieses Konservierungsmittel gilt als gut hautverträglich, sodass die nach der Rasur verbleibenden Reste auch einer Rasierschaumzusammensetzung, die ein solches Konservierungsmittel enthält, verteilt werden und auf der Haut verbleiben können.

Allerdings wird bevorzugt zur Konservierung Zinklactat (Zinksalz der Milchsäure, auch Zinklactat-Dihydrat) eingesetzt, das antimikrobielle und entzündungshemmende mit feuchtigkeitsspendenden und hautberuhigenden Eigenschaften verbindet und eine sehr gute Hautverträglichkeit aufweist. Zinklactat trägt somit dazu bei, Hautreizungen und Entzündungen nach der Rasur durch Reduktion bzw. Kontrolle der Haut-Mikroorganismen zu vermeiden. Außerdem gestattet die Verwendung von Zinklactat die Produktkennzeichnung "frei von Konservierungsstoffen"; allerdings ist ein Behälter wie eine Sprühdose, ein Ventilbeutelbehälter oder ein luftloser Spender erforderlich, die sicherstellen, dass die eingefüllte Rasierschaumzusammensetzung nach Anbruch nicht mit der Luft oder Haut in Kontakt kommen kann.

Der pH-Wert der Rasierschaumzusammensetzung ist im Beispiel mit Milchsäure auf 5,5 eingestellt. Ein Vorteil von Milchsäure besteht darin, dass sie wie Zinklactat feuchtigkeitsspendende Eigenschaften und konservierende Eigenschaften aufweist. Alternativ zu Milchsäure kann Zitronensäure eingesetzt werden. Damit entspricht der pH-Wert der Rasierschaumzusammensetzung einem durchschnittlichen pH-Wert der Haut, der zwischen 4,1 und 5,8 schwankt. Um den Säureschutzmantel der Haut zu erhalten, kann der pH-Wert einer Rasierschaumzusammensetzung, die auch für empfindliche und/oder trockene Haut geeignet ist, vorzugsweise in diesem Bereich zwischen 4 und 6 eingestellt werden. Ferner ist beispielsweise zum Einsatz von Rokonsal^{™} BS als Konservierungsstoff ein pH < 5,4 erforderlich, der entsprechend durch Zugabe von Milchsäure oder Zitronensäure eingestellt werden kann. Andererseits kann die Rasierschaumzusammensetzung ggf. mit höheren, auch alkalischen pH-Werten bis 8 bereitgestellt werden, um das Aufweichen der Haare zu unterstützen und so die Rasur bei dichterem oder längerem Haarwuchs erleichtern zu können. Zur Einstellung eines alkalischen pH-Werts kann ein basischer pH-Regulierer wie z. B. Natronlauge oder ggf. auch Arginin verwendet werden.

## Patentansprüche

1. Seifenfreie Rasierschaumzusammensetzung, ausgebildet zum Auftragen auf eine behaarte Hautpartie, zum Aufweichen von Haaren auf der behaarten Hautpartie, zur Bereitstellung eines Rasierklingen-Gleitfilms auf der Hautpartie,
**dadurch gekennzeichnet, dass**
die Rasierschaumzusammensetzung zumindest
- 1,5 bis 5 Gew.-% zumindest eines Fettalkoholpolyglycolethers aus einem Fettalkohol mit 9 bis 25 Kohlenstoffatomen und 3 bis 30 Ethylenoxideinheiten,
- 2,5 bis 9 Gew.-% einer Ölkomponente, die acyclische Kohlenwasserstoffe mit 11 bis 45 Kohlenstoffatomen aufweist,
- 1,5 bis 5 Gew.-% zumindest eines Fettalkohols mit 12 bis 25 Kohlenstoffatomen und
- einen die 100 Gew.-% ergänzenden Anteil an Wasser aufweist.

2. Rasierschaumzusammensetzung nach Anspruch 1, **dadurch gekennzeichnet, dass** die Rasierschaumzusammensetzung
- 2 bis 4 Gew.-%, insbesondere 3 Gew.-%, des zumindest einen Fettalkoholpolyglycolethers, und/oder
- 3,5 bis 7 Gew.-%, insbesondere 5 Gew.-%, der Ölkomponente, und/oder
- 2 bis 4 Gew.-%, insbesondere 3 Gew.-%, des zumindest einen Fettalkohols, aufweist.

3. Rasierschaumzusammensetzung nach Anspruch 1 oder 2, **dadurch gekennzeichnet, dass**
die Rasierschaumzusammensetzung ferner zumindest einen Pflanzenextrakt in einem zur Hautpflege wirksamen Anteil aufweist,
wobei der zumindest eine Pflanzenextrakt bevorzugt ein Pflanzenextrakt aus Aloe-Vera ist, und der zur Hautpflege wirksame Anteil des Pflanzenextrakts aus Aloe-Vera, der aus Aloe-Vera-Gel getrocknet als Pulver vorliegt, in der Rasierschaumzusammensetzung 8 bis 15 Gew.-%, bevorzugt 9 bis 13 Gew.-%, insbesondere 10 bis 12 Gew.-%, beträgt.

4. Rasierschaumzusammensetzung nach zumindest einem der Ansprüche 1 bis 3, **dadurch gekennzeichnet, dass**
die Rasierschaumzusammensetzung ferner
- 0,5 bis 1,5 Gew.-%, bevorzugt 0,7 bis 1,3 Gew.-%, insbesondere 1 Gew.-%, Panthenol und/oder
- 1 bis 3 Gew.-%, bevorzugt 1,5 bis 2,2 Gew.-%, insbesondere 2 Gew.-%, Zinklactat oder 0,05 bis 1 Gew.-%, bevorzugt 0,1 bis 0,9 Gew.-%, eines Konservierungsmittels, das aus einer Gruppe, die Natriumbenzoat, Kaliumsorbat, Benzylalkohol, Benzoesäure und Mischungen davon umfasst, ausgewählt ist, und/oder
- einen zur Einstellung eines bestimmten pH-Werts der Rasierschaumzusammensetzung in einem Bereich von 4 bis 8, bevorzugt von 5 bis 5,5, erforderlichen Anteil zumindest eines pH-Wert-Regulierers, der aus einer Gruppe ausgewählt ist, die Zitronensäure und Milchsäure zur pH-Wert-Verringerung und Natriumhydroxyd zur pH-Wert-Erhöhung umfasst, und/oder
- bis zu 0,5 Gew.-% zumindest eines Duftstoffs
aufweist.

5. Rasierschaumzusammensetzung nach Anspruch 4, **dadurch gekennzeichnet, dass** die Rasierschaumzusammensetzung aus
- 1,5 bis 5 Gew.-% des zumindest einen Fettalkoholpolyglycolethers,
- 2,5 bis 9 Gew.-% der Ölkomponente,
- 1,5 bis 5 Gew.-% des zumindest einen Fettalkohols,
- 8 bis 15 Gew.-% des zumindest einen Pflanzenextrakts,
- 0,5 bis 1,5 Gew.-% Panthenol,
- 1 bis 3 Gew.-% Zinklactat oder 0,05 bis 1 Gew.-% des Konservierungsmittels,
- einem zur Einstellung eines bestimmten pH-Werts der Rasierschaumzusammensetzung in einem Bereich von 4 bis 8, bevorzugt von 5 bis 5,5, erforderlichen Anteil zumindest eines pH-Wert-Regulierers, der aus einer Gruppe ausgewählt ist, die Zitronensäure und Milchsäure zur pH-Wert-Verringerung und Natriumhydroxyd zur pH-Wert-Erhöhung umfasst,
- bis zu 0,5 Gew.-% des zumindest eines Duftstoffs
und dem die 100 Gew.-% ergänzenden Anteil an Wasser besteht.

6. Rasierschaumzusammensetzung nach zumindest einem der Ansprüche 1 bis 5, **dadurch gekennzeichnet, dass**
die Ölkomponente aus
- verzweigten, acyclischen, gesättigten Kohlenwasserstoffe mit 15 bis 45 Kohlenstoffatomen, bevorzugt acyclischen Terpen-Kohlenwasserstoffen, besonders bevorzugt Sesquiterpenen oder Triterpenen, insbesondere Squalan, oder
- paraffinischen Kohlenwasserstoffen mit 15 bis 45, bevorzugt 18 bis 32, Kohlenstoffatomen, insbesondere Paraffinöl Perliquidum, oder
- einer Mischung aus paraffinischen Kohlenwasserstoffen mit 11 bis 25, bevorzugt 15 bis 19, Kohlenstoffatomen, und verzweigten, acyclischen Kohlenwasserstoffen mit 15 bis 45 Kohlenstoffatomen, bevorzugt acyclischen Terpen-Kohlenwasserstoffen, besonders bevorzugt Sesquiterpenen oder Triterpenen, oder
- einer Mischung aus paraffinischen Kohlenwasserstoffen mit 11 bis 25, bevorzugt 15 bis 19, Kohlenstoffatomen und Fettsäureestern aus Fettsäuren mit 8 bis 20 Kohlenstoffatomen und Fettalkoholen mit 6 bis 25 Kohlenstoffatomen besteht.

7. Rasierschaumzusammensetzung nach zumindest einem der Ansprüche 1 bis 6,
**dadurch gekennzeichnet, dass**
- der zumindest eine Fettalkoholpolyglycolether aus einem Fettalkohol mit 14 bis 22, bevorzugt 16 bis 20, insbesondere 18, Kohlenstoffatomen und mit 4 bis 30, bevorzugt 10 bis 30, besonders bevorzugt 15 bis 25, insbesondere 21, Ethylenoxideinheiten besteht, und/oder
- der zumindest eine Fettalkohol 14 bis 22 Kohlenstoffatome, insbesondere 16 bis 18 Kohlenstoffatome aufweist.

8. Verfahren zur Herstellung einer seifenfreien Rasierschaumzusammensetzung nach zumindest einem der Ansprüche 1 bis 7, umfassend die Schritte
- Zusammengeben des zumindest einen Fettalkoholpolyglycolethers, der Ölkomponente und des zumindest einen Fettalkohols, Erhitzen auf eine erste Temperatur in einem Bereich von 75 bis 85 °C, insbesondere 80 °C, und Durchmischen des zumindest einen Fettalkoholpolyglycolethers, der Ölkomponente und des zumindest einen Fettalkohols durch Rühren,
- Erhitzen des Wassers auf die erste Temperatur, und in das erhitze Wasser Zugeben der erhitzen Mischung aus dem zumindest einen Fettalkoholpolyglycolethers, der zumindest einen Ölkomponente und des zumindest einen Fettalkohols,
- Homogenisieren zu einer Emulsion und Abkühlen der Emulsion.

9. Verfahren nach Anspruch 8, wobei das Abkühlen der Emulsion die Schritte umfasst
- Abkühlen der Emulsion auf eine zweite Temperatur in einem Bereich von 35 bis 45 °C, insbesondere auf 40 °C,
- Zugeben des zumindest einen Pflanzenextrakts, des Panthenols, des Zinklactats oder des Konservierungsmittels, des zumindest einen pH-Wert-Regulierers und des zumindest einen Duftstoffs zu der abgekühlten Emulsion,
- erneut Homogenisieren und Abkühlen auf eine dritte Temperatur in einem Bereich der Raumtemperatur, bevorzugt in einem Bereich von 15 bis 25 °C, insbesondere auf 20 °C.

10. Verfahren nach Anspruch 8 oder 9, wobei das Homogenisieren unter Unterdruck im Grobvakuum, bevorzugt bei einem Druck im Bereich um 500 hPa, erfolgt.

11. Verfahren nach zumindest einem der Ansprüche 8 bis 10, umfassend die Schritte
- Abfüllen einer vorbestimmten Menge der Rasierschaumzusammensetzung in einen Behälter, bevorzugt in einen Behälter, der aus einer Gruppe ausgewählt ist, die eine Sprühdose, einen Ventilbeutelbehälter und einen luftlosen Pumpspender auf-weist.

12. Verfahren nach Anspruch 11, umfassend die Schritte
- Verbringen von Treibgas in die Sprühdose nach dem Abfüllen der vorbestimmten Menge der Rasierschaumzusammensetzung in die Sprühdose, wobei das Treibgas aus der Gruppe ausgewählt ist, die Propan, Butan, Isopentan, Isobutan, Stickstoff, Kohlensäure und Mischungen davon umfasst, wobei das Treibgas einen Anteil von bis zu 5 Gew.-%, bevorzugt 3 bis 4 Gew.-% der treibgashaltigen Rasierschaumzusammensetzung ausmacht, oder
- Anordnen eines Ventilbeutels in dem Ventilbeutelbehälter und Verbringen eines Treibmittels in den Ventilbeutelbehälter vor dem Einfüllen der vorbestimmten Menge der Rasierschaumzusammensetzung in den Ventilbeutel, wobei das Treibmittel bevorzugt ein nicht brennbares Treibgas ist, oder
- luftdicht Anordnen einer Kolbenplatte in dem Behälter eines luftlosen Pumpspenders vor oder nach dem Abfüllen der vorbestimmten Menge der Rasierschaumzusammensetzung in den Behälter.

13. Verwendung einer seifenfreien Rasierschaumzusammensetzung nach zumindest einem der Ansprüche 1 bis 7 zum Rasieren einer behaarten Hautpartie, umfassend das Aufschäumen der Rasierschaumzusammensetzung unter Auftragen auf die behaarte Hautpartie, aufweichen Lassen von Haaren der behaarten Hautpartie und Bereitstellen des Rasierklingen-Gleitfilms auf der Hautpartie.
